(19) Europäisches Patentamt
European Patent Office
Office européen des brevets

(11) Publication number: 0 189 247
A1

# (12) EUROPEAN PATENT APPLICATION

(21) Application number: 86300064.2

(22) Date of filing: 07.01.86

(51) Int. Cl.⁴: **C 07 C 43/13**, C 07 C 69/14, C 07 C 41/03, C 07 C 67/26

(30) Priority: 09.01.85 GB 8500503

(43) Date of publication of application: 30.07.86 Bulletin 86/31

(84) Designated Contracting States: BE DE FR GB IT NL

(71) Applicant: **BP Chemicals Limited, Belgrave House 76 Buckingham Palace Road, London, SW1W 0SU (GB)**

(72) Inventor: **Alderson, Geoffrey W. BP Chemicals Limited, Belgrave House 76 Buckingham Palace Road, London, SW1W 0SU (GB)**
Inventor: **Green, Michael James, The british Petroleum Co. p.l.c., Chertsey Road, Sunbury-on-Thames Middlesex, TW16 7LN (GB)**

(74) Representative: **Fawcett, Richard Fennelly et al, BP INTERNATIONAL LIMITED Patents Division Chertsey Road, Sunbury-on-Thames Middlesex, TW16 7LN (GB)**

(54) **Process for the preparation of glycol ethers and glycol ether esters.**

(57) A process for producing glycol ethers by reacting an alcohol with an epoxide is provided. The reaction is carried out in the presence of an anion exchange resin containing one or more amino groups. In an embodiment of the process, a carboxylic acid ester is used as an additional reactant, whence a mixture of a glycol ether and glycol ether ester is produced. The process can be used to make propylene glycol monoethyl ether from propylene oxide and ethanol or a mixture of propylene glycol monoethyl ether and the acetate ester of propylene glycol monoethyl ether by reacting propylene oxide, ethanol and ethyl acetate.

EP 0 189 247 A1

# PROCESS FOR THE PREPARATION OF GLYCOL ETHERS AND GLYCOL ETHER ESTERS

In one aspect, this invention relates to a process for the preparation of glycol ethers while in another aspect, this invention relates to the preparation of mixtures of glycol ethers and glycol ether esters.

The preparation of glycol ethers by the reaction of an epoxide such as ethylene oxide or propylene oxide with an alcohol using either acid or alkaline catalysts is known. However, the use of anion exchange resins for the production of glycol ethers or mixtures of glycol ethers and glycol ether esters has not been previously reported.

It has now been found that alcohols can be reacted with epoxides in the presence of anion exchange resins to produce glycol ethers in high conversions and high selectivities. Moreover, glycol ethers can be coproduced with glycol ether esters by reacting alcohols, epoxides and carboxylic acid esters in the presence of anion exchange resins. Use of such anion exchange resins has the secondary advantage that such materials are easily separated from the reaction products.

Accordingly, the present invention provides a process for the production of a glycol ether comprising reacting an alcohol and an epoxide at elevated temperature in the presence of an anion exchange resin containing one or more amino groups.

In further embodiment, the present invention provides a process for the production of a mixture of a glycol ether and a glycol ether ester comprising reacting an alcohol, an epoxide, and a carboxylic

acid ester at elevated temperature in the presence of an anion exchange resin containing one or more amino groups.

The alcohol reactants useful in the present invention are $C_1$ to $C_{20}$ monohydric aliphatic alcohols. Preferred alcohols are $C_1$ to $C_6$ saturated alcohols with $C_1$ to $C_4$ saturated alcohols (i.e. methanol, ethanol and the isomeric propanols and butanols) being most preferred.

The epoxide reactants useful herein are compounds of the formula:

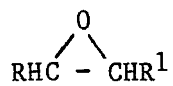

(I)

where R and $R^1$ are independently hydrogen, a $C_1$ to $C_{20}$ substituted or unsubstituted alkyl or a $C_2$ to $C_{20}$ alkenyl radical. Preferably, R is hydrogen and $R^1$ is hydrogen or a $C_1$ to $C_6$ alkyl or $C_2$ to $C_6$ alkenyl radical. $C_2$ to $C_4$ alkylene oxides are the most preferred epoxide reactants, e.g. ethylene oxide, propylene oxide, epichlorohydrin and the like.

Generally, the anion exchange resin useful herein are natural or synthetic resins containing one or more active amino groups which are insoluble in the reactants/products. The amino group can be a primary, secondary or tertiary amino or a substituted secondary or tertiary amino group. Preferred are tertiary or substituted tertiary amino groups. By the term amino group is meant a group containing trivalent nitrogen.

The resin can be any of the well known synthetic resins such as functionalised polyacrylics e.g. polyacrylic acid, polyesters, polyolefins e.g. polystyrenes or copolymers of polystryene and divinyl benzene and the like. Useful resins can also include natural resins such as vegetable-derived mixtures or exudations of carboxylic acids, essential oils and terpenes.

The preparation of glycol ethers suitably occurs by contacting the alcohol and epoxide reactants in the liquid phase in the presence of the anion exchange resin at elevated temperatures. This can occur for example, batchwise, in which the reactants and anion exchange resin are contacted in a sealed vessel, or continuously

where the reactants are pumped over a bed of the anion exchange resin. The process conditions of the inventive reaction can vary widely. Typically, the temperature may range from 50° to 200°C, preferably from 75° to 125°C. The pressure can also vary widely with pressures ranging from 1 to 50 bar found suitable. Optionally, a diluent gas such as nitrogen can be employed.

The mole ratio of alcohol to epoxide can range from 1/1 to 20/1 with mole ratios of 5/1 to 10/1 being preferred.

In a further embodiment of the process described above a mixture of glycol ethers and glycol ether esters can be produced. Such embodiments comprise reacting an alcohol, epoxide and carboxylic acid ester in the presence of the anion exchange resin. Suitable carboxylic acid esters include the esters of monocarboxylic and dicarboxylic acid esters preferably those in which the parent carboxylic acid has from one to twenty carbon atoms. Examples of carboxylic acid esters include methyl acetate, ethyl acetate, methyl propionate, diethylsuccinate, butyl acetate and the like. The carboxylic acid ester is preferably added in amounts corresponding to between 1 and 50 mole % of the alcohol used.

The process comprising the further embodiment is preferably carried out at a temperature in the range 50° to 200°C, preferably 75° to 125°C and at a pressure in the range 1 to 50 bars. The product of this process, which comprises a glycol ether and a glycol ether ester can be separated into its constituents by distillation.

Either process of this invention can be conducted in a continuous, semi-continuous or batch mode operation.

The process for producing glycol ethers and glycol ether esters is illustrated with reference to the following Examples. However, the scope of this invention should not be limited by the Examples contained herein but includes equivalent embodiments, variations and modifications.

Example 1

Glycol ethers were prepared by contacting ethanol and propylene oxide over a commercially obtained anion exchange resin

(Duolite A375). The anion exchange resin has the following properties:

Properties

| | |
|---|---|
| Matrix | Macroporous polyacrylic |
| Functional groups | Tertiary amines |
| Physical form | Cream-coloured beads |
| Ionic form as shipped | Free base |
| Specific gravity | About 1.1 |
| Shipping weight | About 730 g/l |
| Particle size | 0.3 - 1.2 mm |
| Moisture retention capacity | 58-63% (free base form) |
| Total exchange capacity | 1.6 eq/l (free base) |
| Apparent pK | About 9.5 |
| Maximum reversible swelling | 25% (free base to $Cl^-$) |
| Chemical strength | Insoluble in solutions of acids, bases and common solvents. |

A feed containing ethanol and propylene oxide (mole ratio of 10/1) was passed over 20 mls of catalyst in a continuous micro-reactor at 98°C and 26 bar for 30 minutes. Analysis of the reaction products by gas chromatography showed the conversion of propylene oxide was 85% and the selectivity to propylene glycol monoethylether was 85% of which 96% was the secondary isomer (methoxy group on the primary carbon atom). The conversion rate and product distribution remained steady over a test period of 2 hours once the steady state reaction conditions had been achieved.

Example 2

Glycol ether esters and glycol ethers were prepared in accordance with Example 1 except that ethyl acetate was added to the feed. The mole ratio of ethanol/propylene oxide/ethyl acetate was 5/5/1 and the temperature was between 90 and 100°C at 25 bar. Analysis of the reaction product by GC showed that the major products of reaction were propylene glycol monoethyl ether and propylene glycol monoethyl ether acetate ester.

Claims:

1. A process for the production of a glycol ether comprising reacting an alcohol and an epoxide at elevated temperature in the presence of an anion exchange resin containing one or more amino groups.

2. A process as claimed in Claim 1 wherein the anion exchange resin is selected from a polyacrylic, polyester or polyolefin resin functionalised with a tertiary or substituted tertiary amino group.

3. A process as claimed in Claim 1 wherein the alcohol is a $C_1$ to $C_6$ saturated alcohol and the epoxide is a $C_2$ to $C_4$ alkylene oxide.

4. A process as claimed in Claim 2 wherein the alcohol is either methanol or ethanol and the epoxide is either ethylene oxide or propylene oxide.

5. A process for the production of a mixture of a glycol ether and a glycol ether ester comprising reacting an alcohol, an epoxide and a carboxylic acid ester at elevated temperature in the presence of an anion exchange resin containing one or more amino groups.

6. A process as claimed in Claim 5 wherein the anion exchange resin is selected from a polyacrylic, polyester or polyolefin resin functionalised with a tertiary or substituted tertiary amino group.

7. A process as claimed in Claim 5 wherein the carboxylic acid ester is one derived from a carboxylic acid having from one to twenty carbon atoms.

8. A process as claimed in Claim 7 wherein the carboxylic acid ester is selected from methyl acetate, ethyl acetate, diethyl succinate and butyl acetate.

0189247

European Patent Office

# EUROPEAN SEARCH REPORT

Application number

EP 86 30 0064

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-2 104 502 (VYSOKA SKOLA CHEMICKO-TECHNOLOGICKA) * Claims; examples 5,6 * | 1,3 | C 07 C 43/13<br>C 07 C 69/14<br>C 07 C 41/03<br>C 07 C 67/26 |
| A | | 2,4 | |
| | --- | | |
| A | BE-A- 657 517 (CELANESE) * Abstract * | 5-8 | |
| | --- | | |
| A,P | EP-A-0 140 545 (BP CHEMICALS) * Claims; examples * | 1-8 | |
| | ----- | | |

| TECHNICAL FIELDS SEARCHED (Int. Cl.4) |
|---|
| C 07 C 41/00<br>C 07 C 43/00<br>C 07 C 67/00<br>C 07 C 69/00 |

The present search report has been drawn up for all claims

| Place of search | Date of completion of the search | Examiner |
|---|---|---|
| THE HAGUE | 16-04-1986 | WRIGHT M.W. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document